# EUROPEAN PATENT SPECIFICATION

(11) **EP 4 072 626 B1**
(45) Date of publication and mention of the grant of the patent: **18.02.2026**
(21) Application number: 20820961.9
(22) Date of filing: 11.12.2020
(51) Int. Cl.: A61M 5/20, A61M 5/142

(54) **MEDICAL DEVICE COMPRISING AN ALARM GENERATOR TAKING INTO ACCOUNT ENVIRONMENTAL SIGNALS**
MEDIZINISCHES GERÄT MIT ALARMGENERATOR WELCHER UMGEBUNGSSIGNALE MIT EINBEZIEHT
DISPOSITIF MÉDICAL AVEC GÉNÉRATEUR D'ALARME TENANT EN COMPTE DES SIGNAUX D'ENVIRONNEMENT

(30) Priority: 12.12.2019 EP 19306635
(43) Date of publication of application: 19.10.2022
(73) Proprietor: SANOFI, 75017 Paris (FR)
(72) Inventor: HELMER, Michael, 65926 Frankfurt am Main (DE)
(74) Representative: Rose, Kathryn Clare
(86) International application number: PCT/EP2020/085706
(87) International publication number: WO 2021/116376

(56) References cited:
- WO-A1-2019/086527
- US-A1- 2006 097 884
- US-A1- 2016 051 749
- US-A1- 2017 068 799
- US-A1- 2018 036 466
- US-B2- 10 328 204
- US-B2- 8 487 758

## Description

### Field

The present disclosure relates to an apparatus for providing an audible alert to a user and, in particular, to an apparatus for providing an audible alert relating to the status of a drug delivery device.

### Background

A variety of diseases exists that require regular treatment by injection of a medicament by a medical device. Such injection can be performed by using injection devices, which are applied either by medical personnel or by patients themselves. As an example, type-1 and type-2 diabetes can be treated by patients themselves by injection of insulin doses, for example, once or several times per day. For instance, a pre-filled disposable insulin pen can be used as an injection device. Alternatively, a re-usable pen may be used. A reusable pen allows replacement of an empty medicament cartridge by a new one. Either pen may come with a set of one-way needles that are replaced before each use. The insulin dose to be injected can then, for instance, be manually selected at the insulin pen by turning a dosage knob and the actual dose can be observed from a dose window or display of the insulin pen. The dose is then injected by inserting the needle into a suited skin portion and pressing an injection button of the insulin pen.

Administering an injection, however, is a process which presents a number of risks and challenges for users and healthcare professionals, both mental and physical. Patients may forget to administer the medicament in accordance with their dosage regime or they may find it difficult to determine from the drug delivery device information related to a condition and/or use of the drug delivery device.

To try and assist users, it has been described to provide a drug delivery device, for instance an injection device, having an acoustic signal generator, which is configured to produce acoustic signals relating to the operating status of an injection device as feedback to the user. It has also been described to provide a haptic signal generator to provide haptic feedback, for instance by means of vibration, as a means of feedback to the user.

Audible feedbacks are, however, difficult to validate against international standards, particularly where signals are generated by mechanical action. In addition, there is no guarantee that a user will acknowledge or understand the feedback provided.

Medical devices including audible alarms are disclosed in US 10,328,204 B2, US 2016/051749 A1, US 2018/036466 A1, US 8,487,758 B2. US 2017/068799 A1 and WO 2019/086527 A1 discloses systems including a pen injection device, and a mobile communication unit adapted to communicate with the pen and having an indicating means including an audible alert.

Thus, there remains a need for an improved device that can provide effective feedback to a user, and an improved medical device in combination with a management device that can provide effective feedback to prevent false handling of the medical device and/or to keep track of a dosage regime.

It is an aim to provide an apparatus that provides further patient support and facilitates an improved user experience when managing a dosage regime involving a medical device. It is also an aim to provide a system in which the apparatus operates in combination with a second device to provide such patient support. For instance, to assist the user to more reliably or more accurately, or more consistently, or more usefully determine when the device should be used and/or determine the operational status of the drug delivery device or to improve another aspect of the process of managing the dosage regime.

### Summary

According to an aspect of the present invention, there is provided a medicament injection device according to claim 1.

This device may increase the reliability of the feedback alert output from the device as the signal generated is specifically customised to the surrounding environmental conditions in a way that improves the likelihood that the alert will be recognised and understood. This in turn can assist a user to more reliably manage their medical condition.

The medical device may further comprise a vibration element configured to generate a haptic feedback. The alert may further comprise the haptic feedback.

This is advantageous as it provides an additional mechanism by which the alert can be selected to take into account the surrounding environmental conditions. This improves the likelihood that the alert will be recognised. This may be particularly beneficial to those who have hearing impediments, where an additional type of alert improves the likelihood that the alert will be recognised.

Alternatively, the acoustic properties of the alert may be additionally selected based on predetermined settings. This enables the type of alert to be customised to account for both ambient environmental conditions and the specific requirement of the user to improve the likelihood that the alert will be acknowledged and understood.

The predetermined settings may comprise any one or more of feedback preferences, acoustic preferences, hearing limitations, visual limitations, and haptic limitations. This enables a variety of limitations to be taken into account when selecting an appropriate alert.

The medical device may further comprise a photoelectric element configured to detect ambient light levels, such that the acoustic properties of the alert are additionally selected based on the detected ambient light levels.

This provides an additional mechanism by which the alert can be customised to the surrounding environmental conditions in a way that improves the likelihood that the alert will be recognised and understood.

The controller of the medical device may be configured to control the acoustic signal generator to generate the alert having a frequency spectrum, pitch or tone that is selected based on the detected environmental acoustic signals.

Additionally or alternatively, the controller may be configured to control the acoustic signal generator to generate the alert within a frequency band that is selected based on the detected environmental acoustic signals.

Additionally, the controller may be configured to control the acoustic signal generator to generate the alert with an amplitude within each of multiple frequency bands selected based on the detected environmental acoustic signals.

Additionally or alternatively, the controller may be configured to control the acoustic signal generator to generate the alert at a volume that is selected based on the detected environmental acoustic signals.

The ability of the controller to select specific acoustic properties ensures that the alert can be customised to the surrounding environmental conditions in a way that improves the likelihood that the alert will be recognised and understood.

The acoustic properties of the alert may be additionally selected based on a status of the medical device.

This ensures that the alert is associated with operation of the medical device such that a user is able to more reliably manage their medical condition.

The medical device comprises a second acoustic signal generator, where the controller is further configured to control the second acoustic signal generator to generate a reference noise arranged to eliminate ambient noise, in addition to the alert.

This is advantageous as it provides a noise cancelling mechanism that enables a user to better hear the alert produced by the primary acoustic signal generator, thereby improving the efficacy of the alert.

The medical device may be any one of a drug delivery device, a supplemental device provided with a part for coupling the supplemental device to a drug delivery device.

The medical device is a medicament injection device. The medicament injection device may be an injection pen or a supplemental device provided with a part for coupling the supplemental device to an injection pen.

According to another aspect of the present invention, there is provided a feedback system according to claim 9.

This system is advantageous as the combination of first and second devices in a system enables the components for generating the alert to be distributed between the devices such that their operation can be achieved more effectively. This can help the user to more reliably manage their dosage regime.

The first device and the second device may be a medicament injection device, and the other one of the first device and the second device is a mobile device or a controller device.

The first device may be an injection pen or a supplemental device provided with a part for coupling the supplemental device to an injection pen, and the second device may be a mobile device.

The first device may be any one of a medical device, a mobile device and a controller device, and the second device may be a different one of a medical device, a mobile device and a controller device.

The first device may be a drug delivery device or a supplemental device provided with a part for coupling the supplemental device to a drug delivery device, and the second device may be a mobile device.

The first device or the second device may further comprise a vibration element configured to generate a haptic feedback, and the alert may further comprise the haptic feedback.

The acoustic properties of the alert may be additionally selected based on predetermined settings.

The first device or the second device may further comprise a photoelectric element configured to detect ambient light levels. The acoustic properties of the alert are additionally selected based on the detected ambient light levels.

The first device and the second device may further comprise a wireless unit, and the second device is configured to receive status information from the first device, and the controller may be further configured to, in response to receiving the status information, generate an alert having acoustic properties that are additionally selected based on the status information.

Additionally, there is disclosed a method of generating an alert at a medical device comprising: detecting ambient sounds; and generating an alert having acoustic properties that are selected based on the detected environmental conditions.

The alert may further comprise a haptic feedback.

The acoustic properties of the alert may be additionally selected based on predetermined settings. The predetermined settings may comprise any one or more of feedback preferences, acoustic preferences, hearing limitations, visual limitations, and haptic limitations.

The method may further comprise receiving information relating to the status of the medical device; and generating an alert having acoustic properties that are additionally selected based on the status information.

Additionally, there is disclosed a computer program comprising machine readable instructions that when executed by a controller, causes the controller to perform a method of generating an alert at a medical device.

Embodiments of the invention are set out in the dependent claims. In the following, embodiments that do not fall within the scope of the claims relate to exemplary embodiments of the disclosure that are not covered by the claimed invention.

Embodiments of the present disclosure will now be described, by way of example only, with reference to the accompanying drawings.

### Brief Description of the Figures

In the Figures:
Figure 1 is a schematic view showing the internal components of a mobile device having an apparatus according to embodiments of the present disclosure.
Figure 2 is a flow chart showing operation of the apparatus according to embodiments of the present disclosure.
Figure 3 is a flow chart showing selection of predetermined settings, in particular User Group settings, according to embodiments of the present disclosure.
Figure 4 is a flow chart showing selection of predetermined settings, in particular User Specified settings, according to embodiments of the present disclosure.
Figure 5 is a schematic view illustrating various audio settings of Figure 4 selectable according to embodiments of the present disclosure.
Figure 6 is a schematic view illustrating the various device operations of Figure 4 selectable according to embodiments of the present disclosure.
Figure 7 is a flow chart showing operation of the apparatus according to embodiments of the present disclosure.
Figure 8 is a flow chart showing operation of the apparatus according to embodiments of the present disclosure.
Figure 9 is a schematic view illustrating a mobile device wirelessly communicating with a drug delivery device according to other embodiments of the present disclosure. In Figure 9A the acoustic signal generator is integrally formed with an injection device. In Figure 9B the acoustic signal generator is integrally formed with a supplemental device releasably attached to the injection device. In Figure 9 the injection device is an injection pen.
Figure 10 is a flow chart showing operation of the apparatus according to embodiments of the present disclosure.

### Detailed Description

In the following, embodiments of the present disclosure will be described with reference to an apparatus, namely an automatic sound generator, for providing an audible alert that is implemented as a medical device. In particular, the medical device is described as a drug delivery device. The present disclosure is not, however, limited to such application and the apparatus may equally well be implemented in another medical device or an alternative device, such as a mobile device.

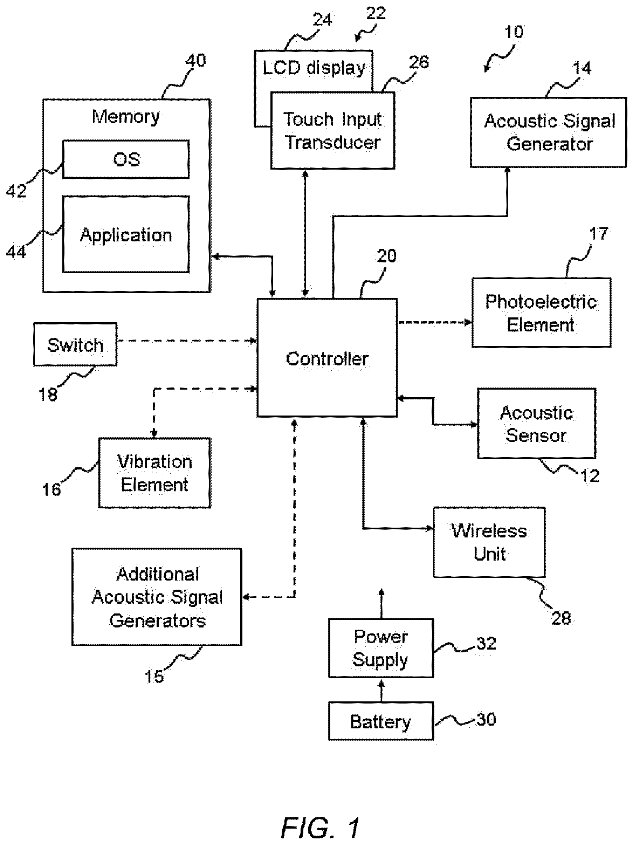

According to a first group of embodiments the apparatus is implemented in a medical device 10. The medical device 10 is a drug delivery device, such as an injection device 1. In particular, the medical device 10 may be an injection device 1 in the form of an injection pen. Figure 1 is a schematic view showing the internal components of the medical device 10.

The medical device 10 includes a plurality of components in combination with the automatic sound generator. The medical device 10 comprises a display 22 (for instance an LCD, TFT (thin film transistor), OLED (organic light emitting diode), ePaper). The display 22 may be a touch sensitive display having a display part 24 and a tactile interface part 26. The display part 24 may be a resistive touch screen or capacitive touch screen of any kind. Alternatively, in some examples the display part 24 may not be a touch screen and may instead be a liquid crystal display (LCD).

The medical device 10 also includes a communications interface 28, for instance a wireless communication interface, such as a Bluetooth interface. The medical device 10 also houses a battery 30 to power the medical device 10 by a power supply 32.

The medical device 10 also includes a controller 20. The controller 20 controls operation of the other hardware components of the medical device 10. The controller 20 and other hardware components may be connected via a system bus (not shown). Each hardware component may be connected to the system bus either directly or via an interface.

The medical device 10 comprises a memory 40, i.e. a working or volatile memory, such as Random Access Memory (RAM), and a non-volatile memory. The volatile memory may be a RAM of any type, for example Static RAM (SRAM), Dynamic RAM (DRAM) or a Flash memory. The controller 20 may access RAM in order to process data and may control the storage of data in the memory 40. The non-volatile memory may be of any kind, such as a Read Only Memory (ROM), a flash memory and a magnetic drive memory. The non-volatile memory stores an operating system 42 and one or more software modules 44, as well as storing data files and associated metadata. The software modules 44 may be distinct, discrete applications that may be provided in the medical device 10 on manufacture or may be downloaded into the medical device 10 by a user, for instance from an application market place or application store.

The controller 20 is configured to send and receive signals to and from the other components in order to control operation of the other components. For example, the controller 20 controls the display of content on display 22 and receives signals as a result of user inputs from the tactile interface 26.

The controller 20 operates under control of the operating system 42. The operating system 42 may comprise code relating to hardware such as the display 22 and the communications interface 28, as well as the basic operation of the medical device 10. The operating system 42 may also cause activation of other software modules stored in the memory 40.

The medical device 10 includes an acoustic sensor 12. The controller 20 controls the acoustic sensor 12. The acoustic sensor 12 is configured to detect environmental acoustic signals 60. The acoustic sensor 12 may be any suitable type of acoustic sensor capable of detecting environmental acoustic signals 60. For instance, the acoustic sensor 12 may be a microphone, such as a moving coil or dynamic microphone, a condenser microphone or a piezoelectric microphone.

The acoustic sensor 12 is configured to detect a variety of acoustic signals derived from different environments, for instance, acoustic signals at a train station, an airport or other type of transport hub, or at a restaurant. These are just examples and the acoustic sensor 12 may equally well detect acoustic signals from other environments. In other words, the acoustic sensor 12 is able to detect the surrounding environmental conditions as characterised by the acoustic signals emitted in those environments. The acoustic sensor 12 or the controller 20 may equally well be responsible for processing and analysing the detected acoustic signals.

The medical device 10 includes an acoustic signal generator 14. The controller 20 also controls the acoustic signal generator 14. The acoustic signal generator 14 may be any suitable type of electric acoustic signal generator capable of emitting a synthesised audio signal. For instance, the acoustic signal generator 14 may be a speaker,

The controller 20 controls the acoustic signal generator 14 to emit an audible alert to a user. The audio alert provides an acoustic feedback 203a. The audible alert may be any suitable form of alert, for instance, a beep or a click or a piece of music. The alert may be a pre-recorded sound. Alternatively, the alert may be synthesised by the acoustic signal generator 14 under control of the controller 20. The pre-recorded sound may be stored in the memory 40 of the medical device 10.

The medical device 10 may also include a photoelectric element 17 (or photosensor), although this is not essential. The controller 20 is configured to control the photoelectric element 17. The photoelectric element 17 may be of any suitable kind that is capable of detecting ambient light levels. For instance, the photoelectric element 17 is a light sensor, such as a photodiode, a phototransistor, or a photoresistor. The light sensor 17 is configured to detect light signals or ambient light levels in the surrounding environment. In other words, the light sensor 17 is configured to detect the surrounding environmental conditions as characterised by the level of light detected in those environments. The light sensor 17 or the controller 20 may equally well be responsible for processing and analysing the detected ambient light levels.

The medical device 10 may also include a vibration element 16, although this is not essential. The controller 20 also controls the vibration element 16. The vibration element 16 is of any suitable kind capable of emitting a tactile or haptic feedback 203b. For instance, the vibration element 16 may be a mechanical or an electronic device (e.g. piezoelectric or moving coil).

The vibration element 16 is configured to provide a structure-borne sound and not merely a tactile feedback. The vibration element 16 may work in combination with the acoustic signal generator 14 to provide a structure-borne sound, in addition to the acoustic signal generated by the acoustic signal generator 14. The vibration element 16 may, for instance, provide an additional buzzing sound, as an acoustical feedback. Alternatively, the vibration element 16 may provide a silent vibration to provide only a tactile feedback. The silent vibration may be selected as an option, for instance, when the medical device 10 is configured to operate in a private mode or silent mode.

The medical device 10 may also include a switch 18, although this is not essential. The controller 20 controls the switch 18. The switch 18 may be of any suitable kind, for instance, a mechanical switch (such as a slider, rocker or push button switch) or an electronic switch (a touch sensor), or a software-implemented switch (e.g. activated through a graphical user interface provided on a touch-sensitive display).

The switch 18 is configured to control whether the acoustic signal is output at the medical device 10. In other words, the switch 18 is configured to act as a mute switch. When the switch 18 is in a first position, for instance, the acoustic signal generator 14 of the medical device 10 is able to produce an audible alert. When the switch 18 is in a second position, for instance, the acoustic signal generator 14 of the medical device 10 is muted and is prevented from producing an audible signal. In one example, the first position is an ON position and the second position is an OFF position.

The switch 18 may also be configured to control the output of the vibration element 16. The switch 18 may control the output of the vibration element 16 instead of, or in combination with, the acoustic signal generator 14. When the switch 18 is in a first position, for instance, the vibration element 16 generates an alert (a structure-borne sound) and the acoustic signal generator 14 generates an alert. When the switch 18 is in a second position, for instance, the vibration element 16 generates an alert but the acoustic signal generator 14 is not able to generate an alert. In other words, the switch 18 may control the medical device 10 to operate in a vibration only mode. This may be a private mode or silent mode. Alternatively, the switch 18 may be configured to control the degree of feedback (intensity of the alert) emitted by the vibration element 16. For instance, the switch 18 may control the vibration element 16 to generate a 'soft vibration' alert that is not as powerful as an alert generated under normal operation. The soft vibration alert may represent a private mode.

The acoustic sensor 12 and acoustic signal generator 14 represent components of the automatic sound generator apparatus. The vibration element 16, photoelectric element 17 and the switch 18 are additional, optional, components of the automatic sound generator apparatus. Other suitable components could also be included.

Other standard or optional components of the medical device 10, such as light sources (LEDs) or user input transducers (e.g. button, switch, touch-sensitive), are omitted.

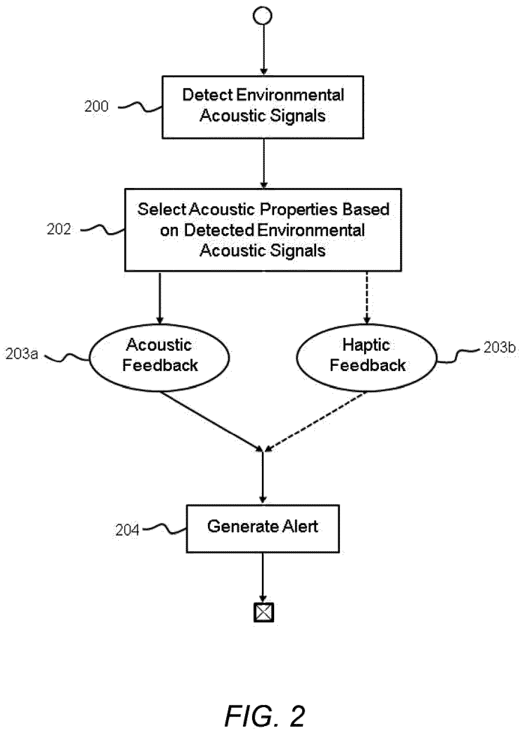

As set out above, the controller 20 controls the acoustic signal generator 14 to generate an alert 50. In the following, the generation of the alert 50 is described in more detail and with reference to Figure 2. Figure 2 is a flow chart illustrating the generation of an alert 50 according to embodiments of the present disclosure.

In Figure 2, the operation starts, for instance, at step 200 where the acoustic sensor 12 detects environmental acoustic signals 60. In step 202, the controller 20 determines the acoustic properties of the acoustic signal that is to be generated and output as an alert 50. An acoustic signal comprises a variety of acoustic properties that are variable to create different sounds. Acoustic properties may include, for instance, a frequency spectrum having multiple frequency bands, pitch, tone, intensity and amplitude. In step 202, the controller 20 selects acoustic properties, such as those described above, based on the environmental conditions detected by the acoustic sensor 12. The acoustic properties selected contributed to the acoustic feedback 203a provided. In step 204, the controller 20 controls the acoustic signal generator 14 to generate an alert 50 having acoustic properties as selected in step 202.

The controller 20 is configured to control the acoustic signal generator 14 to generate the alert 50, for instance, with acoustic properties having a particular frequency spectrum, pitch or tone. In particular, the controller 20 is configured to control the acoustic signal generator 14 to generate the alert 50 having a frequency spectrum, pitch or tone that is selected based on the detected environmental acoustic signals 60. For instance, the alert 50 may be generated within a frequency band that is selected based on the detected environmental acoustic signals 60. In addition, the alert 50 may be generated, for instance, with an amplitude within each of multiple frequency bands selected based on the detected environmental acoustic signals 60. Alternatively, the alert 50 may be generated at a volume that is selected based on the detected environmental acoustic signals 60.

In generating the alert 50, the controller 20 may synthesise a new audio signal according to the acoustic properties selected based on the detected environmental acoustic signals 60. Alternatively, in generating the alert 50, the controller 20 may modify the acoustic properties of a pre-stored audio signal based on the detected environmental acoustic signals 60.

In other words the medical device 10 is capable of producing an alert 50 that is tailored to the ambient environmental conditions. As the acoustic sensor 12 detects environmental acoustic signals 60, the ambient environmental conditions can be assessed. It is possible to determine whether the medical device 10 is located in a loud or busy environment, such as a train station. In such an environment, a multitude of different sounds originate from a variety of sources (e.g. people, trains, Tannoy announcements), each having different acoustic properties. Alternatively, the medical device 10 may be located in a quiet or calm environment, such as a restaurant or a library or a theatre. Accordingly, the way in which the alert 50 is generated can be customised according to the surrounding environmental conditions. If the medical device 10 is at an airport, it may be beneficial for the alert 50 to be characterised by a high frequency band, for instance, in order for the alert 50 to be heard above the background ambient noise. Conversely, if the medical device 10 is in a library then a more discrete alert is required and a mid-range frequency band, for instance, may be selected instead.

Figure 2 also shows that optionally, in some embodiments, the controller may control the vibration element 16 to generate a haptic feedback 203b in combination with the acoustic feedback 203a provided by the acoustic signal generator 14. The haptic feedback 203b may include a structure-borne sound in combination with a tactile feedback. Alternatively, the haptic feedback 203b may only provide a tactile feedback. As set out above, in some embodiments, the configuration of the switch 18 determines the type of haptic feedback 203b provided by the vibration element 16 in combination with, or instead of, the acoustic feedback 203a provided by the acoustic signal generator 14.

In the example described above, the alert 50 is generated based on detected environmental acoustic signals 60. The present disclosure is not, however, limited to such an example and the acoustic properties of the alert 50 may equally well be selected based on other properties.

For instance, according to a second group of embodiments, in addition to the detected environmental acoustic signals 60, the acoustic properties of the alert 50 may be selected based on predetermined settings 70. The predetermined settings 70 may include, for instance, a user group 72 and/or a user specified setting 74. The predetermined settings 70 may be provided on manufacture or may be downloaded, or may be allocated by a user. The predetermined settings 70 may be selected or modified or updated and set by a user via a series of interfaces or menus that can be accessed via the display of the medical device 10. The controller 20 may receive signals as a result of user inputs from the tactile interface 26, for instance.

The predetermined settings 70 may relate to any one or more of (user) feedback preferences, acoustic preferences, hearing limitations, visual limitations, and haptic limitations. Accordingly, the predetermined settings 70 are provided to enable a user to configure the medical device 10 to operate in a way that the acoustic signal generated is tailored to the specific needs of that user. For instance, the predetermined settings 70 configure the medical device 10 such that the characteristics of the alert generated are optimised according to the preferences and physical requirements of the user, such that the likelihood that the user will detect the alert is increased.

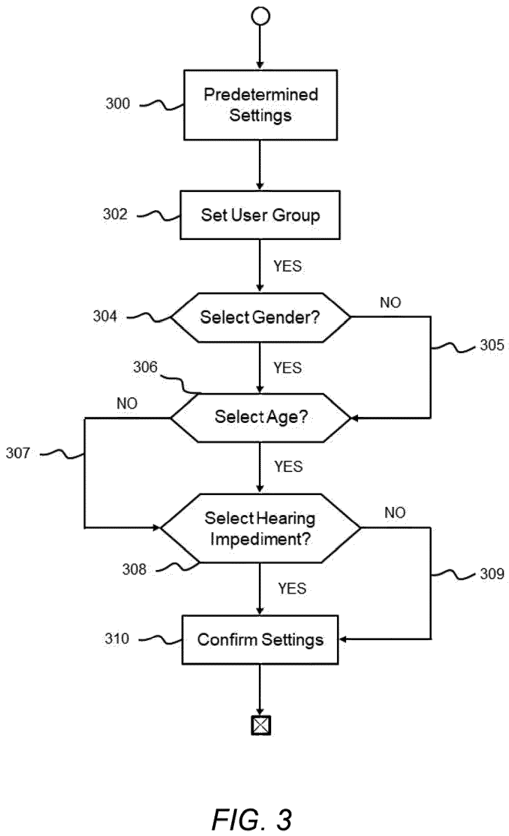

Figure 3 is a flow chart illustrating the selection of predetermined settings 70 relating to a User Group according to embodiments of the present disclosure. The predetermined User Group settings may include one or more of gender, age or hearing impediment. These are just examples and other settings could also be provided. For instance, settings relating to visual impediments or tactile impediments. In Figure 3, the operation starts, for instance, at step 300 where the settings 70 of the medical device 10 are accessed via the display 22 of the medical device 10. In step 302, the user group 62 setting is selected. This selection leads to a series of sub-settings or options that can be selected and set or modified, and stored.

In step 304, the Gender setting is selected, and the appropriate gender, i.e. male or female, is input and stored. Alternatively, in step 305 this setting is not selected. In step 306, the Age setting is selected and the user's age is input and stored. Alternatively, in step 307, this setting is not selected. In step 308, the Hearing Impediment setting is selected. This enables a particular hearing impediment, such as tinnitus, to be selected. Alternatively, or in addition, this setting also enables audio preferences to be selected based on general hearing ability or audio requirements. This could include a variety of audio options, for instance, sounds, melodies, frequency, and loudness. Alternatively, in step 309, this setting is not selected. In step 310, the user group 62 settings are confirmed and stored.

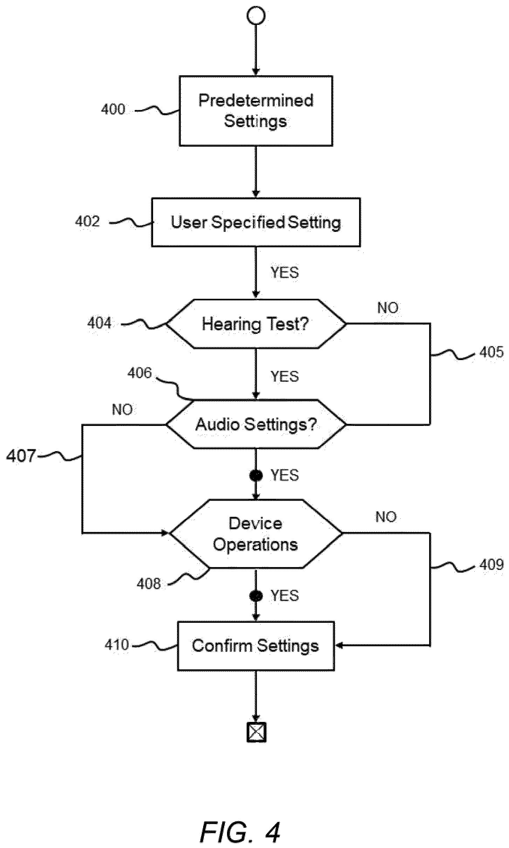

Figure 4 is a flow chart illustrating the selection of predetermined settings 70 relating to a user specified 64 setting according to embodiments of the present disclosure. The predetermined user specified 64 settings may include one or more of a hearing test, audio settings, or device operations. These are just examples and other settings could also be provided. In Figure 4, the operation starts, for instance, at step 400 where the settings 70 of the medical device 10 are accessed via the display 22 of the medical device 10. In step 402, the User Specified setting is selected. This selection leads to a series of sub-settings or options that can be selected and set or modified, and stored. In step 404, the Hearing Test setting is selected. This performs a hearing test that determines the general hearing capabilities of the user. The hearing test may be of any suitable kind that can be performed with the medical device 10. The hearing test may be carried out, for instance, by connecting headphones to the medical device 10. The hearing test may be carried out, for instance, in a variety of environments. The results of the hearing test may be stored. Alternatively, in step 405, this setting is not selected.

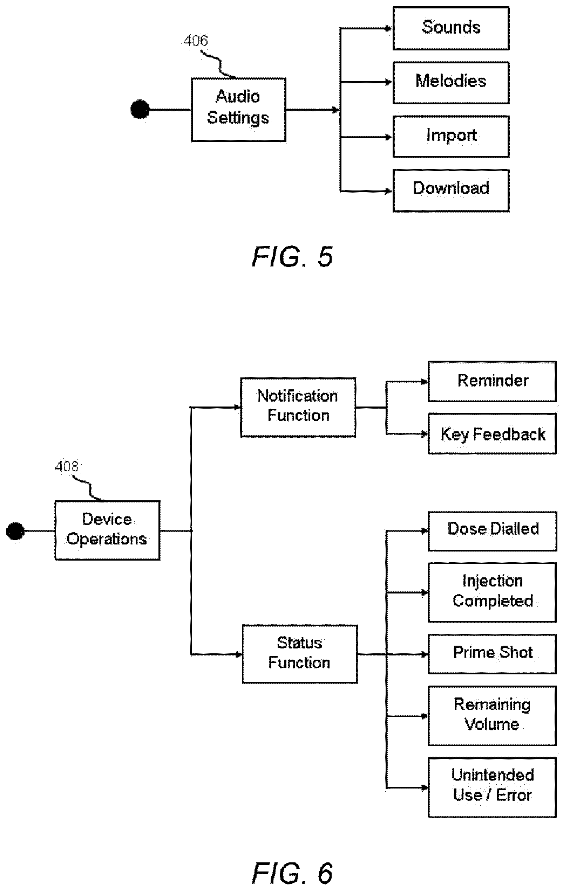

In step 406, the Audio setting is selected. This setting determines the type and style of audio signal to be used in generating the alert 50. Figure 5 is a schematic illustration of examples of such audio settings. For instance, the sound of the alert in the form of, for instance, a chirp or a beep can be selected from pre-stored recordings. In addition, the melody of the audio signal for the alert can be selected from pre-stored recordings. Alternatively, recordings stored in the memory 40 of the medical device 10 can be imported or can be downloaded from an external device, for instance, from the internet via a web browser. Alternatively, in step 407, this setting is not selected.

In step 408, the Device Operations setting is selected. This setting determines which operations are allocated to an alert. Figure 6 is a schematic illustration of examples of such device operations. As shown in Figure 6, the device operations may include a series of functions, for instance, a notification function and a status function.

The notification function may be directed to providing an alert in response to a particular action or operation of the medical device 10. As shown in Figure 6, the notification function may include options such as a Reminder setting and a Key Feedback setting. The reminder setting may, for instance, relate to a clock alarm, an event, or other similar tasks. In one example, the reminder may relate to a user's dosage regime and may be configured to provide an alert, for instance, at a time when the user requires, or is likely to require, a dose of medicament. These are just examples and other options could also be provided. The Key Feedback setting may, for instance, notify the user when an area of the display 22 of the medical device 10 has received an input signal or if a mechanical button provided at the medical device 10 has received a touch input. The device operations setting enables the notification functions to be turned on or off according to audio requirements. These are just examples and other options could also be provided.

As shown in Figure 6, the status function may be directed to providing an alert in relation to, or in response to, the status of the medical device 10. For instance, if the medical device 10 is a drug delivery device, the status function may notify a user of a status of a drug delivery device. The status of the drug delivery device may include options such as, completion of a dialled dose, completion of a delivered dose (e.g. injection completed), completion of a prime shot, a warning as to the amount of medicament remaining, and a warning as to unintended use or an error in use of the drug delivery device. These are just examples, however, and other options and other medical devices could also be provided.

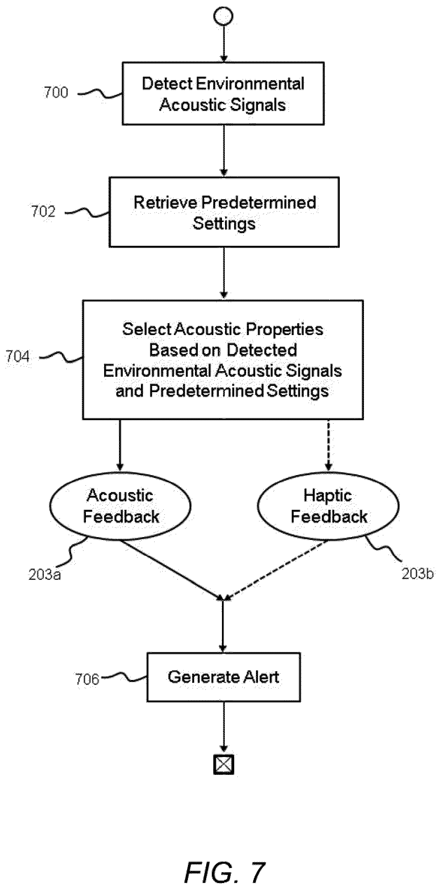

As set out above, the controller 20 controls the acoustic signal generator 14 to generate an alert 50 having acoustic properties based on the detected environmental acoustic signals 60 and additionally based on predetermined settings 70. In the following, the generation of the alert 50 is described in more detail and with reference to Figure 7. Figure 7 is a flow chart illustrating the generation of an alert 50 based on detected environmental acoustic signals 60 and predetermined settings 70.

In Figure 7, the operation starts, for instance, at step 700 where the acoustic sensor 12 detects environmental acoustic signals 60. This is substantially the same as step 200 in relation to Figure 2. In step 702, the controller 20 retrieves the predetermined settings 70 that have been previously selected and set, as described above, from the memory 40 of the medical device 10. In step 704, the controller 20 selects acoustic properties based on the environmental conditions detected by the acoustic sensor 12 and additionally based on the predetermined settings 70. In other words, the acoustic properties of the alert are selected based on a combination of both the detected environmental conditions and the retrieved predetermined settings 70. In step 706, the controller 20 controls the acoustic signal generator 14 to generate an alert 50 having acoustic properties as selected in step 704.

Figure 7 also shows that optionally, in some embodiments, the controller 20 may control the vibration element 16 to generate a haptic feedback 203b in combination with the acoustic feedback 203a provided by the acoustic signal generator 14. This feedback is substantially the same as that described above in relation to Figure 2 and a detailed explanation will not be repeated.

The ability of a user to acknowledge and understand an alert may vary according to gender, age or pre-existing hearing or visual abilities. Thus, the provision of predetermined settings 70 relating to a particular user group, as described above, enables the controller 20 to vary the acoustic properties of an alert based on the settings stored. This is advantageous as the apparatus is operable to produce a customised alert that is tailored, not only to account for ambient environmental conditions, but also to compensate for the particular physiology and/or medical condition of the user.

Similarly, the requirements of each user may vary and therefore it is beneficial for the configuration of the apparatus operate in accordance with those requirements. For instance, it may only be required to provide an alert for some or not all of the operations of the medical device 10. Alternatively, a specific type of alert may be required in association with a particular operation of the medical device 10. In addition, alerts may not be required in relation to the status of a drug delivery device. Alternatively, only some alerts may be required in relation to selected statuses of a drug delivery device.

Thus, the provision of predetermined settings 70 relating to device operations, as described above, enables the controller 20 to vary the acoustic properties of an alert 50 based on the settings stored. This is advantageous as the apparatus is operable to produce a customised alert 50 that is tailored, not only to account for ambient environmental conditions, but also to meet the operating requirements of the medical device 10. This acts to improve the likelihood that a user will acknowledge and understand the nature of an alert 50.

In the examples described above, the alert is generated based on detected environmental acoustic signals 60 or detected environmental acoustic signals 60 and predetermined settings 70. The present disclosure is not, however, limited to these examples and the acoustic properties of the alert 50 may equally well be selected based on other properties.

According to a third group of embodiments, for instance, in addition to the detected environmental acoustic signals 60, the acoustic properties of the alert 50 may be selected based on the light signals detected by the photoelectric element 18.

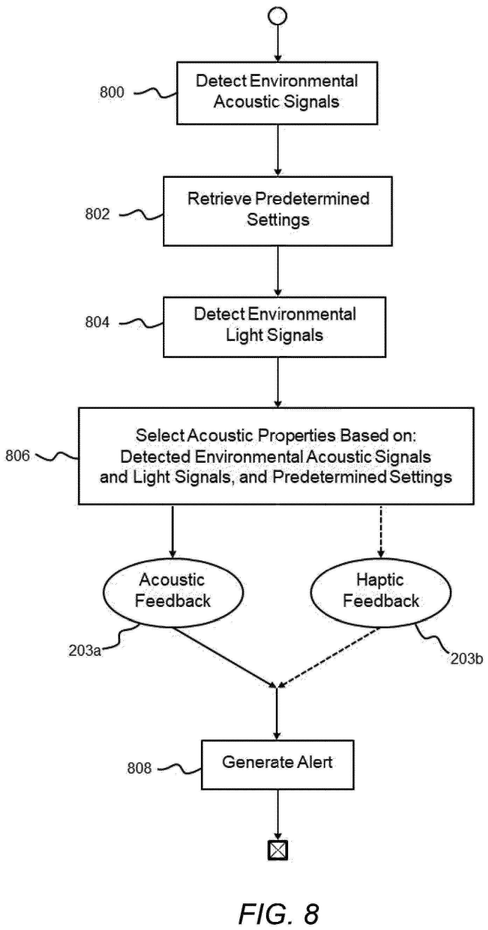

Figure 8 is a flow chart illustrating the generation of an alert 50 according to the third group of embodiments of the present disclosure. As set out above, in addition to the detected environmental acoustic signals 60 and the predetermined settings 70, the acoustic properties of the alert 50 are selected based on the detected light levels or ambient environmental light conditions.

In Figure 8, step 800 and step 802 are substantially the same as steps 700 and step 702, respectively, as disclosed above in relation to Figure 7 and a detailed explanation will not be repeated. Briefly, however, in step 800, the acoustic sensor 12 detects environmental acoustic signals 60 and in step 802, the controller 20 retrieves the predetermined settings 70. In step 804, the light sensor 17 detects light signals.

In step 806, the controller 20 determines the acoustic properties of the acoustic signal that is to be generated and output as an alert 50. Specifically, in step 806, the controller 20 selects acoustic properties, based on the environmental conditions detected by the acoustic sensor 12 and the light sensor 17 and the predetermined settings 70. The acoustic properties are substantially the same as those described above in relation to Figure 2, step 202, such that a detailed explanation will not be repeated. In step 808, the controller 20 controls the acoustic signal generator 14 to generate an alert 50 having acoustic properties as selected in step 806.

Figure 8 also shows that optionally, in some embodiments, the controller 20 may control the vibration element 16 to generate a haptic feedback 203b in combination with the acoustic feedback 203a provided by the acoustic signal generator 14. This feedback is substantially the same as that described above in relation to Figure 2 and a detailed explanation will not be repeated.

In the examples described above, the apparatus is described as a medical device 10. In particular, the medical device 10 is an injection device 1, such as an injection pen. The present disclosure is not, however, limited to such an implementation.

In accordance with a fourth group of embodiments of the present disclosure one or more components of the apparatus may be implemented in a first device and other one or more components of the apparatus may be implemented in a second device. These components may include one or more of the acoustic sensor 12, acoustic signal generator 14, vibration element 16, photoelectric element 17 or switch 18, which may be included in the automatic sound generator apparatus. However, other components could also be included.

The first device and second device are separate, discrete devices. The operation of the first device and second device in combination forms a feedback system that provides an audible alert. The first and second devices may be any suitable device having at least a controller, a memory and wireless communication capabilities. These components may be substantially the same as those described and shown in relation to Figure 1. Other components may be included according to the type of device. These are in addition to the one or more components of the automatic sound generator. The second device is able to send and/or receive data to and/or from the first device in order to control the operation of the automatic sound generator. The data may include information relating to the first device and/or the automatic sound generator. For instance, the first device may be any one of a medical device (e.g. a drug delivery device, such as an injection pen or a supplemental device with a part for coupling the supplemental device to a drug delivery device, such as an injection pen), a mobile device and a controller device, and the second device may be a different one of a medical device, a mobile device and a controller device.

In the following, the fourth group of embodiments will be described with reference to the acoustic signal generator 14 being implemented in a first device. The present disclosure is not, however, limited to such an application and various combinations of the one or more components could be envisaged in each device. For instance, more than one of the components of the automatic sound generator may equally well be included in either the first device or the second device. Alternatively, all the components of the automatic sound generator could be included in one device and the operation of the automatic sound generator is controlled by the other device.

In the following example, the first device is a medical device 10. In particular, the medical device 10 is a drug delivery device, for instance an injection device 1. In particular, the injection device 1 is an injection pen. In the following example, the second device is a mobile device 100, for instance a mobile phone.

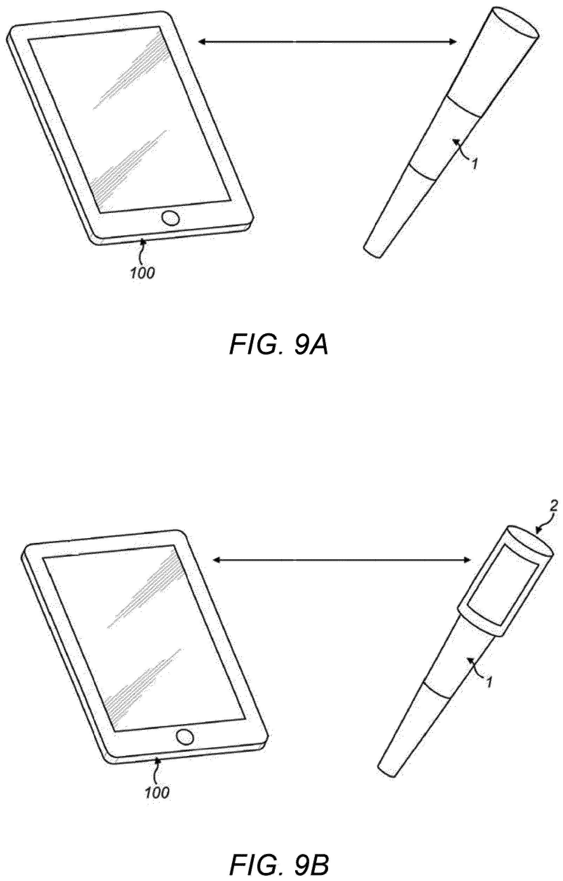

Figure 9 shows a schematic drawing of the mobile device 100 operable with an exemplary drug delivery device according to embodiments of the present disclosure. Figure 9 shows the drug delivery device as an injection device 1 in the form of an injection pen. Figure 9A shows an example in which the acoustic signal generator 14 is integrally formed within the injection device 1. Figure 9B shows an example in which the acoustic signal generator 14 is implemented in a supplemental device 2 that is releasably attached to the injection device 1. The other one or more components of the automatic sound generator are provided in the mobile device 100.

For instance, the drug delivery device is configured to record information relating to a condition and/or use of the drug delivery device and to transmit that information to the mobile device 100. The information may, for instance, relate to the status of the drug delivery device. As shown in Figure 6, the status information may notify a user of, for instance, completion of a dialled dose, completion of a delivered dose (e.g. injection completed), completion of a prime show, a warning as to the amount of medicament remaining, and a warning as to unintended use or an error in use of the drug delivery device. These are just examples, however, and other options could also be provided.

As such, the drug delivery device may further include, for instance, at least one or more sensors for providing information indicative of how the drug delivery device is used. The controller 20 is configured to analyse that use information, and the wireless unit 28 is configured to transmit that use information to the mobile device 100 and the memory 40 is configured to storing the use information. The drug delivery device may, for instance, be an injection device 1.

As shown in Figure 9, the mobile device 100 is configured to receive status information from the drug delivery device via the communications interface of the mobile device 100.

In the examples described above, the alert is generated based on detected environmental acoustic signals 60 or environmental acoustic signals 60 and predetermined settings 70 and/or detected light signals. The present disclosure is not, however, limited to these examples and the acoustic properties of the alert 50 may equally well be selected based on other properties.

In accordance with the fourth group of embodiments, the acoustic properties of the alert 50 are selected based on the detected environmental acoustic signals 60 and additionally based on status information received from a drug delivery device.

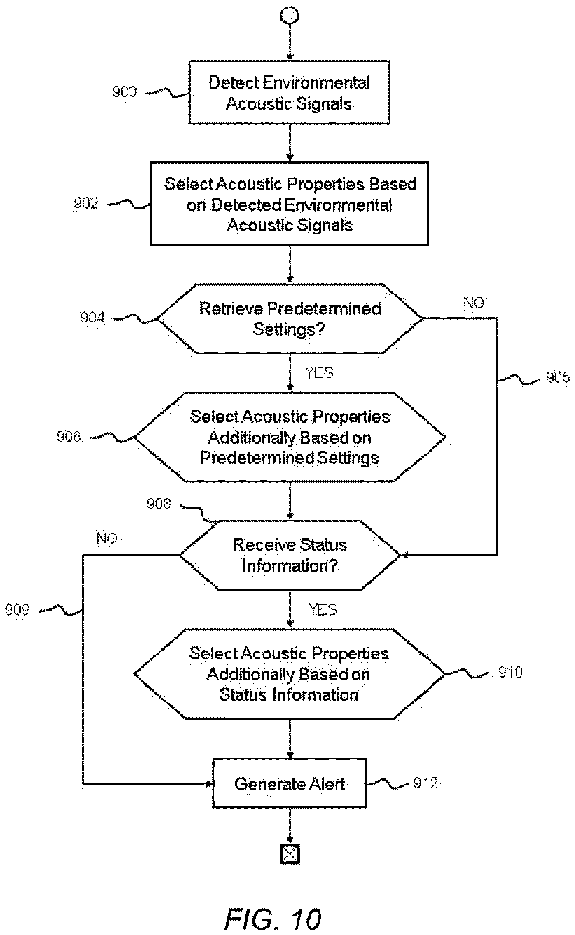

In the following, the generation of the alert 50 is described in more detail and with reference to Figure 10. Figure 11 is a flow chart illustrating the generation of an alert 50 based on detected environmental acoustic signals 60 and predetermined settings 70 and/or status information received from a drug delivery device.

In Figure 10, the operation starts, for instance, at step 900 at the mobile device 100, where the acoustic sensor 12 detects environmental acoustic signals 60. In step 902, the controller 20 selects acoustic properties based on the environmental conditions detected by the acoustic sensor 12. In step 904, the controller 20 retrieves the predetermined settings 70 that have been previously selected and set, as described above, from the memory 40 of the mobile device 100. In step 906, the controller 20 selects acoustic properties additionally based on the predetermined settings 70. In step 908, the controller 20 receives via the communications interface 28 status information relating to the status of the drug delivery device, from the injection device 1 or a supplementary device 2 associated with the drug delivery device (Figure 9). In step 910, in response to the receiving the status information from the drug delivery device, the controller 20 selects acoustic properties additionally based on the status information. In step 912, the controller 20 controls the acoustic signal generator 14 at the medical device 10 to generate an alert 50 having the acoustic properties selected in steps 902, 906 and 910.

Alternatively, in step 905, if none of the predetermined settings 70 have been set, then the operation may proceed to step 908. In this instance, at step 912, the controller 20 controls the acoustic signal generator 14 at the medical device 10 to generate an alert 50 having the acoustic properties selected in steps 902 and 910.

Alternatively, in step 909, if status information is not received from a drug delivery device, it is still possible to generate an alert in response to an operation of the medical device 10 or the mobile device 100. As described above, the medical device 10 includes many operations that may require an alert. Thus, in this instance, at step 912, the controller 20 controls the acoustic signal generator 14 at the medical device 10 to generate an alert 50 having the acoustic properties selected in steps 902 and 906.

Alternatively, if none of the predetermined settings 70 have been set and status information is not received from a drug delivery device, then at step 912, the controller 20 controls the acoustic signal generator 14 to generate an alert 50 having the acoustic properties selected in step 902. That is the controller 20 controls the acoustic signal generator 14 to generate an alert 50 having acoustic properties that are selected based on the detected environmental acoustic signals 60. This is substantially the same as the steps shown in Figure 2.

The fourth group of embodiments is directed to an apparatus that is able to generate an alert 50 in response to the status of a drug delivery device, whilst taking into account the surrounding environmental conditions. The apparatus is also able to customise those alerts according to specific user requirements, as defined by the predetermined settings 70. This is advantageous as it improves the likelihood that a user will acknowledge and understand an alert 50. This will assist the user in more reliably and more accurately determining when the device should be used and/or the operational status of the drug delivery device and ultimately improve implementation of the required dosage regime.

Although not shown in Figure 10, optionally, in some embodiments, the vibration element 16 may generate a haptic feedback 203b in combination with the acoustic feedback 203a provided by the acoustic signal generator 14. This feedback is substantially the same as that described above in relation to Figure 2 and a detailed explanation will not be repeated. Similarly, although not disclosed in Figure 10, the acoustic properties may additionally be selected based on detected light signals, as shown in Figure 8.

Various alternatives and modifications will be apparent to the skilled person. Some such variations and modifications will now be described.

For instance, although the apparatus has been described as a medical device and in particular, a drug delivery device, the present disclosure is not, however, limited to such an application and the medical device could equally well be an infusion device, an injector device, or a pump device. Alternatively, the apparatus could be a mobile device such as a mobile phone, a PDA or a tablet computer of any kind. In the case of a mobile phone, the mobile phone may be a smartphone.

Although the medical device 10 is described as having one acoustic signal generator 14, the apparatus may equally well include a second or a plurality of acoustic signal generators 15, in addition to the first or primary acoustic signal generator 14. The additional one or more acoustic signal generator 15 may have the same or different capabilities to the primary acoustic signal generator 14. According to the claimed invention, the medical device 10 includes a second acoustic signal generator 15.

The second acoustic signal generator 15 is for eliminating or reducing environmental sound and noise. The second acoustic signal generator 15 is thereby configured to act as a noise cancelling mechanism. In this way, the user can better hear the feedback sound produced by the primary acoustic signal generator 14.

The second acoustic signal generator 15 is configured to capture environmental acoustic signals 60. This may be achieved automatically. The additional second acoustic signal generator 15 is also configured to generate a noise eliminating or noise reducing acoustic signal. The second acoustic signal generator 15 may be configured to emit the sound in multiple directions. The second acoustic signal generator 15 may thereby provide a surround sound effect. The operation of the second acoustic signal generator 15 may be controlled by the user through an independent predetermined setting. Where there is a plurality of additional acoustic signal generators 15, each may be configured to eliminate or reduce specific individual environmental acoustic signals 60.

Similarly, although the acoustic sensor 12 and the photoelectric element 17 are described as operating under the control of the controller 20, these components may alternatively operate automatically to detect environmental signals when the apparatus is turned on.

In addition, the switch 18 can be configured to produce various combinations of options with respect to the vibration element 16, the acoustic signal generator 14 and the intensity of the alert generated by the vibration element 16.

Although hearing impediments are used as an example of a predetermined setting 70, settings relating to visual or tactile impediments could equally well be provided. These setting enable a particular visual or physical impediment, such as tunnel vision, to be selected. This enables audio and/or vibration preferences to be selected based on general visual and tactile ability or requirements determined from the settings.

Although specific combinations are described above, it will be evident that the acoustic properties selected based on the detected environmental acoustic signals 60 may additionally be selected based on any combination of the predetermined settings 70, the light signals and the status of the medical device 10.

The terms "drug" or "medicament" are used synonymously herein and describe a pharmaceutical formulation containing one or more active pharmaceutical ingredients or pharmaceutically acceptable salts or solvates thereof, and optionally a pharmaceutically acceptable carrier. An active pharmaceutical ingredient ("API"), in the broadest terms, is a chemical structure that has a biological effect on humans or animals. In pharmacology, a drug or medicament is used in the treatment, cure, prevention, or diagnosis of disease or used to otherwise enhance physical or mental well-being. A drug or medicament may be used for a limited duration, or on a regular basis for chronic disorders.

As described below, a drug or medicament can include at least one API, or combinations thereof, in various types of formulations, for the treatment of one or more diseases. Examples of API may include small molecules having a molecular weight of 500 Da or less; polypeptides, peptides and proteins (e.g., hormones, growth factors, antibodies, antibody fragments, and enzymes); carbohydrates and polysaccharides; and nucleic acids, double or single stranded DNA (including naked and cDNA), RNA, antisense nucleic acids such as antisense DNA and RNA, small interfering RNA (siRNA), ribozymes, genes, and oligonucleotides. Nucleic acids may be incorporated into molecular delivery systems such as vectors, plasmids, or liposomes. Mixtures of one or more drugs are also contemplated.

The drug or medicament may be contained in a primary package or "drug container" adapted for use with a drug delivery device. The drug container may be, e.g., a cartridge, syringe, reservoir, or other solid or flexible vessel configured to provide a suitable chamber for storage (e.g., short- or long-term storage) of one or more drugs. For example, in some instances, the chamber may be designed to store a drug for at least one day (e.g., 1 to at least 30 days). In some instances, the chamber may be designed to store a drug for about 1 month to about 2 years. Storage may occur at room temperature (e.g., about 20°C), or refrigerated temperatures (e.g., from about - 4°C to about 4°C). In some instances, the drug container may be or may include a dual-chamber cartridge configured to store two or more components of the pharmaceutical formulation to-be-administered (e.g., an API and a diluent, or two different drugs) separately, one in each chamber. In such instances, the two chambers of the dual-chamber cartridge may be configured to allow mixing between the two or more components prior to and/or during dispensing into the human or animal body. For example, the two chambers may be configured such that they are in fluid communication with each other (e.g., by way of a conduit between the two chambers) and allow mixing of the two components when desired by a user prior to dispensing. Alternatively or in addition, the two chambers may be configured to allow mixing as the components are being dispensed into the human or animal body.

The drugs or medicaments contained in the drug delivery devices as described herein can be used for the treatment and/or prophylaxis of many different types of medical disorders. Examples of disorders include, e.g., diabetes mellitus or complications associated with diabetes mellitus such as diabetic retinopathy, thromboembolism disorders such as deep vein or pulmonary thromboembolism. Further examples of disorders are acute coronary syndrome (ACS), angina, myocardial infarction, cancer, macular degeneration, inflammation, hay fever, atherosclerosis and/or rheumatoid arthritis. Examples of APIs and drugs are those as described in handbooks such as Rote Liste 2014, for example, without limitation, main groups 12 (antidiabetic drugs) or 86 (oncology drugs), and Merck Index, 15th edition.

Examples of APIs for the treatment and/or prophylaxis of type 1 or type 2 diabetes mellitus or complications associated with type 1 or type 2 diabetes mellitus include an insulin, e.g., human insulin, or a human insulin analogue or derivative, a glucagon-like peptide (GLP-1), GLP-1 analogues or GLP-1 receptor agonists, or an analogue or derivative thereof, a dipeptidyl peptidase-4 (DPP4) inhibitor, or a pharmaceutically acceptable salt or solvate thereof, or any mixture thereof. As used herein, the terms "analogue" and "derivative" refers to a polypeptide which has a molecular structure which formally can be derived from the structure of a naturally occurring peptide, for example that of human insulin, by deleting and/or exchanging at least one amino acid residue occurring in the naturally occurring peptide and/or by adding at least one amino acid residue. The added and/or exchanged amino acid residue can either be codable amino acid residues or other naturally occurring residues or purely synthetic amino acid residues. Insulin analogues are also referred to as "insulin receptor ligands". In particular, the term "derivative" refers to a polypeptide which has a molecular structure which formally can be derived from the structure of a naturally occurring peptide, for example that of human insulin, in which one or more organic substituent (e.g. a fatty acid) is bound to one or more of the amino acids. Optionally, one or more amino acids occurring in the naturally occurring peptide may have been deleted and/or replaced by other amino acids, including non-codeable amino acids, or amino acids, including non-codeable, have been added to the naturally occurring peptide.

Examples of insulin analogues are Gly(A21), Arg(B31), Arg(B32) human insulin (insulin glargine); Lys(B3), Glu(B29) human insulin (insulin glulisine); Lys(B28), Pro(B29) human insulin (insulin lispro); Asp(B28) human insulin (insulin aspart); human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Examples of insulin derivatives are, for example, B29-N-myristoyl-des(B30) human insulin, Lys(B29) (N- tetradecanoyl)-des(B30) human insulin (insulin detemir, Levemir^{®}); B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-gamma-glutamyl)-des(B30) human insulin, B29-N-omega-carboxypentadecanoyl-gamma-L-glutamyl-des(B30) human insulin (insulin degludec, Tresiba^{®}); B29-N-(N-lithocholyl-gamma-glutamyl)-des(B30) human insulin; B29-N-(ω-carboxyheptadecanoyl)-des(B30) human insulin and B29-N-(w-carboxyheptadecanoyl) human insulin.

Examples of GLP-1, GLP-1 analogues and GLP-1 receptor agonists are, for example, Lixisenatide (Lyxumia^{®}), Exenatide (Exendin-4, Byetta^{®}, Bydureon^{®}, a 39 amino acid peptide which is produced by the salivary glands of the Gila monster), Liraglutide (Victoza^{®}), Semaglutide, Taspoglutide, Albiglutide (Syncria^{®}), Dulaglutide (Trulicity^{®}), rExendin-4, CJC-1134-PC, PB-1023, TTP-054, Langlenatide / HM-11260C (Efpeglenatide), HM-15211, CM-3, GLP-1 Eligen, ORMD-0901, NN-9423, NN-9709, NN-9924, NN-9926, NN-9927, Nodexen, Viador-GLP-1, CVX-096, ZYOG-1, ZYD-1, GSK-2374697, DA-3091, MAR-701, MAR709, ZP-2929, ZP-3022, ZP-DI-70, TT-401 (Pegapamodtide), BHM-034. MOD-6030, CAM-2036, DA-15864, ARI-2651, ARI-2255, Tirzepatide (LY3298176), Bamadutide (SAR425899), Exenatide-XTEN and Glucagon-Xten.

An example of an oligonucleotide is, for example: mipomersen sodium (Kynamro^{®}), a cholesterol-reducing antisense therapeutic for the treatment of familial hypercholesterolemia or RG012 for the treatment of Alport syndrom.

Examples of DPP4 inhibitors are Linagliptin, Vildagliptin, Sitagliptin, Denagliptin, Saxagliptin, Berberine.

Examples of hormones include hypophysis hormones or hypothalamus hormones or regulatory active peptides and their antagonists, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, and Goserelin.

Examples of polysaccharides include a glucosaminoglycane, a hyaluronic acid, a heparin, a low molecular weight heparin or an ultra-low molecular weight heparin or a derivative thereof, or a sulphated polysaccharide, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium. An example of a hyaluronic acid derivative is Hylan G-F 20 (Synvisc^{®}), a sodium hyaluronate.

The term "antibody", as used herein, refers to an immunoglobulin molecule or an antigen-binding portion thereof. Examples of antigen-binding portions of immunoglobulin molecules include F(ab) and F(ab')2 fragments, which retain the ability to bind antigen. The antibody can be polyclonal, monoclonal, recombinant, chimeric, de-immunized or humanized, fully human, non-human, (e.g., murine), or single chain antibody. In some embodiments, the antibody has effector function and can fix complement. In some embodiments, the antibody has reduced or no ability to bind an Fc receptor. For example, the antibody can be an isotype or subtype, an antibody fragment or mutant, which does not support binding to an Fc receptor, e.g., it has a mutagenized or deleted Fc receptor binding region. The term antibody also includes an antigen-binding molecule based on tetravalent bispecific tandem immunoglobulins (TBTI) and/or a dual variable region antibody-like binding protein having cross-over binding region orientation (CODV).

The terms "fragment" or "antibody fragment" refer to a polypeptide derived from an antibody polypeptide molecule (e.g., an antibody heavy and/or light chain polypeptide) that does not comprise a full-length antibody polypeptide, but that still comprises at least a portion of a full-length antibody polypeptide that is capable of binding to an antigen. Antibody fragments can comprise a cleaved portion of a full length antibody polypeptide, although the term is not limited to such cleaved fragments. Antibody fragments that are useful in the present invention include, for example, Fab fragments, F(ab')2 fragments, scFv (single-chain Fv) fragments, linear antibodies, monospecific or multispecific antibody fragments such as bispecific, trispecific, tetraspecific and multispecific antibodies (e.g., diabodies, triabodies, tetrabodies), monovalent or multivalent antibody fragments such as bivalent, trivalent, tetravalent and multivalent antibodies, minibodies, chelating recombinant antibodies, tribodies or bibodies, intrabodies, nanobodies, small modular immunopharmaceuticals (SMIP), binding-domain immunoglobulin fusion proteins, camelized antibodies, and VHH containing antibodies. Additional examples of antigen-binding antibody fragments are known in the art.

The terms "Complementarity-determining region" or "CDR" refer to short polypeptide sequences within the variable region of both heavy and light chain polypeptides that are primarily responsible for mediating specific antigen recognition. The term "framework region" refers to amino acid sequences within the variable region of both heavy and light chain polypeptides that are not CDR sequences, and are primarily responsible for maintaining correct positioning of the CDR sequences to permit antigen binding. Although the framework regions themselves typically do not directly participate in antigen binding, as is known in the art, certain residues within the framework regions of certain antibodies can directly participate in antigen binding or can affect the ability of one or more amino acids in CDRs to interact with antigen.

Examples of antibodies are anti PCSK-9 mAb (e.g., Alirocumab), anti IL-6 mAb (e.g., Sarilumab), and anti IL-4 mAb (e.g., Dupilumab).

Pharmaceutically acceptable salts of any API described herein are also contemplated for use in a drug or medicament in a drug delivery device. Pharmaceutically acceptable salts are for example acid addition salts and basic salts.

Those of skill in the art will understand that modifications (additions and/or removals) of various components of the APIs, formulations, apparatuses, methods, systems and embodiments described herein may be made without departing from the full scope and spirit of the present invention, which encompass such modifications and any and all equivalents thereof.

An example drug delivery device may involve a needle-based injection system as described in Table 1 of section 5.2 of ISO 11608-1:2014(E). As described in ISO 11608-1:2014(E), needle-based injection systems may be broadly distinguished into multi-dose container systems and single-dose (with partial or full evacuation) container systems. The container may be a replaceable container or an integrated non-replaceable container.

As further described in ISO 11608-1:2014(E), a multi-dose container system may involve a needle-based injection device with a replaceable container. In such a system, each container holds multiple doses, the size of which may be fixed or variable (pre-set by the user). Another multi-dose container system may involve a needle-based injection device with an integrated non-replaceable container. In such a system, each container holds multiple doses, the size of which may be fixed or variable (pre-set by the user).

As further described in ISO 11608-1:2014(E), a single-dose container system may involve a needle-based injection device with a replaceable container. In one example for such a system, each container holds a single dose, whereby the entire deliverable volume is expelled (full evacuation). In a further example, each container holds a single dose, whereby a portion of the deliverable volume is expelled (partial evacuation). As also described in ISO 11608-1:2014(E), a single-dose container system may involve a needle-based injection device with an integrated non-replaceable container. In one example for such a system, each container holds a single dose, whereby the entire deliverable volume is expelled (full evacuation). In a further example, each container holds a single dose, whereby a portion of the deliverable volume is expelled (partial evacuation).

## Claims

1. A medicament injection device comprising:
an acoustic sensor (12) configured to detect environmental acoustic signals (60);
a first acoustic signal generator (14) operable to generate an acoustic signal; and
a controller (20) configured to control the first acoustic signal generator to generate an alert (50) having acoustic properties that are selected based on the detected environmental acoustic signals,
**characterized in that**
the medicament injection device further comprises a second acoustic signal generator (15), and
the controller is further configured to control the second acoustic signal generator to generate a reference noise arranged to eliminate ambient noise, in addition to the alert.

2. The medicament injection device according to claim 1, further comprising a vibration element (16) configured to generate a haptic feedback,
wherein the alert (50) further comprises the haptic feedback.

3. The medicament injection device according to claim 1 or claim 2, wherein the acoustic properties of the alert (50) are additionally selected based on predetermined settings (70), and
optionally the predetermined settings comprise any one or more of feedback preferences, acoustic preferences, hearing limitations, visual limitations, and haptic limitations.

4. The medicament injection device according to any one of the preceding claims, further comprising a photoelectric element (17) configured to detect ambient light levels,
wherein the acoustic properties of the alert are additionally selected based on the detected ambient light levels.

5. The medicament injection device according to any one of the preceding claims, wherein the controller (20) is configured to control the first acoustic signal generator (14) to generate the alert (50) having a frequency spectrum, pitch or tone that is selected based on the detected environmental acoustic signals and/or to generate the alert within a frequency band that is selected based on the detected environmental acoustic signals.

6. The medicament injection device according to any one of the preceding claims, wherein the controller (20) is configured to control the first acoustic signal generator (14) to generate the alert (50) with an amplitude within each of multiple frequency bands selected based on the detected environmental acoustic signals (60), and/or to generate the alert at a volume that is selected based on the detected environmental acoustic signals.

7. The medicament injection device according to any one of the preceding claims, wherein the acoustic properties of the alert are additionally selected based on a status of the medicament injection device.

8. The medicament injection device according to any one of the preceding claims, wherein the medicament injection device is an injection pen or a supplemental device provided with a part for coupling the supplemental device to an injection pen.

9. A feedback system comprising:
a first device (10, 100) comprising a first acoustic signal generator (14) operable to generate an acoustic signal; and
a second device (10, 100) comprising an acoustic sensor (12) configured to detect environmental acoustic signals, and a controller (20) configured to control the first acoustic signal generator of the first device to generate an alert (50) having acoustic properties that are selected based on the detected environmental acoustic signals (60),
wherein one of the first device and the second device is a medicament injection device (10), and the other one of the first device and the second device is a mobile device (100) or a controller device,
**characterized in that**
the first device further comprises a second acoustic signal generator (15), and
wherein the controller is further configured to control the second acoustic signal generator to generate a reference noise arranged to eliminate ambient noise, in addition to the alert.

10. The feedback system according to claim 9, wherein the first device (10) is an injection pen or a supplemental device provided with a part for coupling the supplemental device to an injection pen, and
the second device (100) is a mobile device.

11. The feedback system according to claim 9 or claim 10, wherein either the first device or the second device further comprise a vibration element (16) configured to generate a haptic feedback,
wherein the alert (50) further comprises the haptic feedback.

12. The feedback system according to any one of claims 9 to 11, wherein the acoustic properties of the alert (50) are additionally selected based on predetermined settings (70).

13. The feedback system according to any one of claims 9 to 12, wherein either the first device or the second device further comprise a photoelectric element (17) configured to detect ambient light levels,
wherein the acoustic properties of the alert (50) are additionally selected based on the detected ambient light levels.

14. The feedback system according to any one of claims 9 to 13, wherein the first device and the second device further comprise a wireless unit (28), and
the second device is configured to receive status information from the first device,
wherein the controller (20) of the second device is further configured to, in response to receiving the status information, generate an alert (50) having acoustic properties that are additionally selected based on the status information.

## Patentansprüche

1. Medikamenteninjektionsvorrichtung, umfassend:
einen akustischen Sensor (12), der ausgestaltet ist, um akustische Umgebungssignale (60) zu detektieren;
einen ersten akustischen Signalgenerator (14), der betreibbar ist, um ein akustisches Signal zu generieren; und
eine Steuerung (20), die ausgestaltet ist, um den ersten akustischen Signalgenerator zu steuern, um eine Warnung (50) mit akustischen Eigenschaften zu generieren, die basierend auf den detektierten akustischen Umgebungssignalen ausgewählt werden,
**dadurch gekennzeichnet, dass**
die Medikamenteninjektionsvorrichtung ferner einen zweiten akustischen Signalgenerator (15) umfasst, und
die Steuerung ferner ausgestaltet ist, um den zweiten akustischen Signalgenerator zu steuern, um zusätzlich zu der Warnung ein Referenzgeräusch zu generieren, das dazu vorgesehen ist, Umgebungsgeräusche zu eliminieren.

2. Medikamenteninjektionsvorrichtung nach Anspruch 1, ferner umfassend ein Vibrationselement (16), das ausgestaltet ist, um eine haptische Rückmeldung zu generieren,
wobei die Warnung (50) ferner die haptische Rückmeldung umfasst.

3. Medikamenteninjektionsvorrichtung nach Anspruch 1 oder Anspruch 2, wobei die akustischen Eigenschaften der Warnung (50) zusätzlich basierend auf vorbestimmten Einstellungen (70) ausgewählt werden, und
die vorbestimmten Einstellungen gegebenenfalls eine oder mehrere von Rückmeldepräferenzen, akustischen Präferenzen, Hörbeschränkungen, visuellen Einschränkungen und haptischen Einschränkungen umfassen.

4. Medikamenteninjektionsvorrichtung nach einem der vorhergehenden Ansprüche, ferner umfassend ein photoelektrisches Element (17), das ausgestaltet ist, um Umgebungslichtniveaus zu detektieren,
wobei die akustischen Eigenschaften der Warnung zusätzlich basierend auf den detektierten Umgebungslichtniveaus ausgewählt werden.

5. Medikamenteninjektionsvorrichtung nach einem der vorhergehenden Ansprüche, wobei die Steuerung (20) ausgestaltet ist, um den ersten akustischen Signalgenerator (14) zu steuern, um die Warnung (50) mit einem Frequenzspektrum, einer Tonhöhe oder Klangfarbe zu generieren, das/die basierend auf den detektierten akustischen Umgebungssignalen ausgewählt wird, und/oder die Warnung innerhalb eines Frequenzbands zu generieren, das basierend auf den detektierten akustischen Umgebungssignalen ausgewählt wird.

6. Medikamenteninjektionsvorrichtung nach einem der vorhergehenden Ansprüche, wobei die Steuerung (20) ausgestaltet ist, um den ersten akustischen Signalgenerator (14) zu steuern, um die Warnung (50) mit einer Amplitude innerhalb jedes von mehreren Frequenzbändern zu generieren, die basierend auf den detektierten akustischen Umgebungssignalen (60) ausgewählt sind, und/oder die Warnung mit einer Lautstärke zu generieren, die basierend auf den detektierten akustischen Umgebungssignalen ausgewählt ist.

7. Medikamenteninjektionsvorrichtung nach einem der vorhergehenden Ansprüche, wobei die akustischen Eigenschaften der Warnung zusätzlich basierend auf einem Status der Medikamenteninjektionsvorrichtung ausgewählt werden.

8. Medikamenteninjektionsvorrichtung nach einem der vorhergehenden Ansprüche, wobei die Medikamenteninjektionsvorrichtung ein Injektionspen oder eine Zusatzvorrichtung ist, die mit einem Teil zum Koppeln der Zusatzvorrichtung an einen Injektionspen ausgestattet ist.

9. Rückmeldesystem, umfassend:
eine erste Vorrichtung (10, 100), die einen ersten akustischen Signalgenerator (14) umfasst, der betreibbar ist, um ein akustisches Signal zu generieren; und
eine zweite Vorrichtung (10, 100), die einen akustischen Sensor (12), der ausgestaltet ist, um akustische Umgebungssignale zu detektieren, und eine Steuerung (20) umfasst, die ausgestaltet ist, um den ersten akustischen Signalgenerator der ersten Vorrichtung zu steuern, um eine Warnung (50) mit akustischen Eigenschaften zu generieren, die basierend auf den detektierten akustischen Umgebungssignalen (60) ausgewählt werden,
wobei eine der ersten Vorrichtung und der zweiten Vorrichtung eine Medikamenteninjektionsvorrichtung (10) ist, und die andere der ersten Vorrichtung und der zweiten Vorrichtung eine mobile Vorrichtung (100) oder eine Steuerungsvorrichtung ist,
**dadurch gekennzeichnet, dass**
die erste Vorrichtung ferner einen zweiten akustischen Signalgenerator (15) umfasst, und
wobei die Steuerung ferner ausgestaltet ist, um den zweiten akustischen Signalgenerator zu steuern, um zusätzlich zu der Warnung ein Referenzgeräusch zu generieren, das dazu vorgesehen ist, Umgebungsgeräusche zu eliminieren.

10. Rückmeldesystem nach Anspruch 9, wobei die erste Vorrichtung (10) ein Injektionspen oder eine Zusatzvorrichtung ist, die mit einem Teil zum Koppeln der Zusatzvorrichtung an einen Injektionspen ausgestattet ist, und
die zweite Vorrichtung (100) eine mobile Vorrichtung ist.

11. Rückmeldesystem nach Anspruch 9 oder Anspruch 10, wobei entweder die erste Vorrichtung oder die zweite Vorrichtung ferner ein Vibrationselement (16) umfassen, das ausgestaltet ist, um eine haptische Rückmeldung zu generieren,
wobei die Warnung (50) ferner die haptische Rückmeldung umfasst.

12. Rückmeldesystem nach einem der Ansprüche 9 bis 11, wobei die akustischen Eigenschaften der Warnung (50) zusätzlich basierend auf vorbestimmten Einstellungen (70) ausgewählt werden.

13. Rückmeldesystem nach einem der Ansprüche 9 bis 12, wobei entweder die erste Vorrichtung oder die zweite Vorrichtung ferner ein photoelektrisches Element (17) umfassen, das ausgestaltet ist, um Umgebungslichtniveaus zu detektieren,
wobei die akustischen Eigenschaften der Warnung (50) zusätzlich basierend auf den detektierten Umgebungslichtniveaus ausgewählt werden.

14. Rückmeldesystem nach einem der Ansprüche 9 bis 13, wobei die erste Vorrichtung und die zweite Vorrichtung ferner eine Drahtlos-Einheit (28) umfassen, und
die zweite Vorrichtung ausgestaltet ist, um Statusinformationen von der ersten Vorrichtung zu empfangen,
wobei die Steuerung (20) der zweiten Vorrichtung ferner ausgestaltet ist, um in Reaktion auf Empfangen der Statusinformationen eine Warnung (50) mit akustischen Eigenschaften zu generieren, die zusätzlich basierend auf den Statusinformationen ausgewählt werden.

## Revendications

1. Dispositif d'injection de médicament comprenant :
un capteur acoustique (12) configuré pour détecter des signaux acoustiques environnementaux (60) ;
un premier générateur de signaux acoustiques (14) exploitable pour générer un signal acoustique ; et
un dispositif de commande (20) configuré pour commander le premier générateur de signaux acoustiques afin de générer une alerte (50) ayant des propriétés acoustiques qui sont sélectionnées sur la base des signaux acoustiques environnementaux détectés,
**caractérisé en ce que**
le dispositif d'injection de médicament comprend en outre un second générateur de signaux acoustiques (15), et
le dispositif de commande est en outre configuré pour commander le second générateur de signaux acoustiques afin de générer un bruit de référence conçu pour éliminer le bruit ambiant, en plus de l'alerte.

2. Dispositif d'injection de médicament selon la revendication 1, comprenant en outre un élément vibrant (16) configuré pour générer une rétroaction haptique, dans lequel l'alerte (50) comprend en outre la rétroaction haptique.

3. Dispositif d'injection de médicament selon la revendication 1 ou la revendication 2, dans lequel les propriétés acoustiques de l'alerte (50) sont de plus sélectionnées sur la base de réglages prédéterminés (70), et
facultativement, les réglages prédéterminés comprennent une ou plusieurs parmi préférences de rétroaction, préférences acoustiques, limitations auditives, limitations visuelles et limitations haptiques.

4. Dispositif d'injection de médicament selon l'une quelconque des revendications précédentes, comprenant en outre un élément photoélectrique (17) configuré pour détecter des niveaux de lumière ambiante,
dans lequel les propriétés acoustiques de l'alerte sont de plus sélectionnées sur la base des niveaux de lumière ambiante détectés.

5. Dispositif d'injection de médicament selon l'une quelconque des revendications précédentes, dans lequel le dispositif de commande (20) est configuré pour commander le premier générateur de signaux acoustiques (14) pour générer l'alerte (50) ayant un spectre de fréquences, un ton ou une tonalité qui est sélectionné(e) sur la base des signaux acoustiques environnementaux détectés et/ou pour générer l'alerte dans une bande de fréquences qui est sélectionnée sur la base des signaux acoustiques environnementaux détectés.

6. Dispositif d'injection de médicament selon l'une quelconque des revendications précédentes, dans lequel le dispositif de commande (20) est configuré pour commander le premier générateur de signaux acoustiques (14) pour générer l'alerte (50) avec une amplitude dans chacune des multiples bandes de fréquence sélectionnées sur la base des signaux acoustiques environnementaux détectés (60), et/ou pour générer l'alerte à un volume qui est sélectionné sur la base des signaux acoustiques environnementaux détectés.

7. Dispositif d'injection de médicament selon l'une quelconque des revendications précédentes, dans lequel les propriétés acoustiques de l'alerte sont de plus sélectionnées en fonction d'un état du dispositif d'injection de médicament.

8. Dispositif d'injection de médicament selon l'une quelconque des revendications précédentes, dans lequel le dispositif d'injection de médicament est un stylo d'injection ou un dispositif supplémentaire muni d'une partie pour accoupler le dispositif supplémentaire à un stylo d'injection.

9. Système de rétroaction comprenant :
un premier dispositif (10, 100) comprenant un premier générateur de signaux acoustiques (14) exploitable pour générer un signal acoustique ; et
un second dispositif (10, 100) comprenant un capteur acoustique (12) configuré pour détecter des signaux acoustiques environnementaux, et un dispositif de commande (20) configuré pour commander le premier générateur de signaux acoustiques du premier dispositif pour générer une alerte (50) ayant des propriétés acoustiques qui sont sélectionnées sur la base des signaux acoustiques environnementaux détectés (60),
dans lequel l'un parmi le premier et le second dispositif est un dispositif d'injection de médicament (10), et l'autre parmi le premier et le second dispositif est un dispositif mobile (100) ou un dispositif de commande,
**caractérisé en ce que**
le premier dispositif comprend en outre un second générateur de signaux acoustiques (15), et
dans lequel le dispositif de commande est en outre configuré pour commander le second générateur de signaux acoustiques afin de générer un bruit de référence conçu pour éliminer le bruit ambiant, en plus de l'alerte.

10. Système de rétroaction selon la revendication 9, dans lequel le premier dispositif (10) est un stylo d'injection ou un dispositif supplémentaire muni d'une partie pour accoupler le dispositif supplémentaire à un stylo d'injection, et
le second dispositif (100) est un dispositif mobile.

11. Système de rétroaction selon la revendication 9 ou la revendication 10, dans lequel soit le premier dispositif, soit le second dispositif comprend en outre un élément vibrant (16) configuré pour générer une rétroaction haptique,
dans lequel l'alerte (50) comprend en outre la rétroaction haptique.

12. Système de rétroaction selon l'une quelconque des revendications 9 à 11, dans lequel les propriétés acoustiques de l'alerte (50) sont de plus sélectionnées sur la base de réglages prédéterminés (70).

13. Système de rétroaction selon l'une quelconque des revendications 9 à 12, dans lequel soit le premier dispositif, soit le second dispositif comprend en outre un élément photoélectrique (17) configuré pour détecter des niveaux de lumière ambiante,
dans lequel les propriétés acoustiques de l'alerte (50) sont de plus sélectionnées sur la base des niveaux de lumière ambiante détectés.

14. Système de rétroaction selon l'une quelconque des revendications 9 à 13, dans lequel le premier dispositif et le second dispositif comprennent en outre une unité sans fil (28), et
le second dispositif est configuré pour recevoir des informations d'état du premier dispositif,
dans lequel le dispositif de commande (20) du second dispositif est en outre configuré pour, en réponse à la réception des informations d'état, générer une alerte (50) ayant des propriétés acoustiques qui sont de plus sélectionnées sur la base des informations d'état.
